# EUROPEAN PATENT SPECIFICATION

(11) **EP 3 938 847 B1**
(45) Date of publication and mention of the grant of the patent: **28.01.2026**
(21) Application number: 20721679.7
(22) Date of filing: 12.03.2020
(51) Int. Cl.: G04F 1/00, A61M 37/00, A61K 9/70

(54) **TRANSDERMAL PATCH**
TRANSDERMALES PFLASTER
TIMBRE TRANSDERMIQUE

(30) Priority: 13.03.2019 GB 201903426
(43) Date of publication of application: 19.01.2022
(73) Proprietor: Singh, Arwinder, Catford London SE6 4DP (GB)
(72) Inventor: Singh, Arwinder, Catford London SE6 4DP (GB)
(74) Representative: HGF
(86) International application number: PCT/GB2020/050624
(87) International publication number: WO 2020/183185

(56) References cited:
- EP-B1- 1 283 992
- WO-A1-2019/005852
- US-A- 3 520 124
- US-A- 5 446 705

## Description

### Field

The disclosure relates to the field of transdermal patches, and in particular, although not exclusively, to a transdermal patch timer for a transdermal patch.

### Background

Transdermal patches are used in healthcare industries to deliver an active agent, such as a medicament or therapeutic agent through the skin of a patient. Various transdermal patch structures and configurations are known in the art. A particular benefit of transdermal patches as an active agent delivery mechanism is that the active agent can be delivered to a local site on the patient.

In the field of pain relief, transdermal patches are typically used to deliver a therapeutic agent to the patient. The timescale for delivery using a single transdermal patch may be several days. For example, fentanyl patches may be used to deliver pain relief over a period of three days. Such a treatment plan presents its own difficulties. In particular, it is important to avoid the patient exceeding the recommended dose, which could result in adverse health consequences. Similarly, it is important to ensure that the patient's dosage does not fall below the required level.

Due to the timescales over which therapeutic agents such as fentanyl are delivered, and the condition of the patient receiving therapy, it can be difficult to ensure that the transdermal patches are used as required. For example, patients receiving pain relief may be in a confused state which makes it difficult for them to track the progress of their own treatment. In addition, it is unusual for the same member of care staff, or a family member, to continuously attend to the same patient over a period of several days. Such difficulties mean that the patient's transdermal patch may be replaced too frequently, or not frequently enough. In the event that the transdermal patch is replaced too frequently, the patient may experience adverse consequences as a result of receiving an overdose of active agent. In the event that the transdermal patch is not changed frequently enough, the patient may experience adverse medical consequences due to the lack of active agent being administered for a period of time. Either result is undesirable or harmful.

Time indicators not related to transdermal patches are known, for example US Patent Publication Nos. US 3 520 124 and US 5 446 705. International Publication No. WO2019/00582 discloses a transdermal therapeutic system that includes a timing device. European Patent No. 1283992 discloses a time dependant indicator.

### Summary

According to a first aspect of the disclosure there is provided a transdermal patch timer according to claim 1.

The predetermined period of time may be a period that has elapsed following application of a transdermal patch to a patient.

The attachment mechanism may comprise an adhesive layer configured to adhere the transdermal patch timer to the transdermal patch.

The indicator may be arranged on the transdermal patch timer to be isolated from an active agent reservoir of the transdermal patch. The indicator may be configured to operate independently from the delivery of active agent to the patient. The indicator may comprise a visual indicator configured to undergo a change in appearance over time or after the predetermined period of time has elapsed. The visual indicator may be configured to change colour over time or after the predetermined period of time has elapsed. The indicator may comprise a chemical that changes condition over time or after the predetermined period of time has elapsed. The change of condition may be achieved via a chemical reaction or phase change, for example. The indicator may be a carbon dioxide, or carbonate, indicator. An advantage of such an indicator is that the indication of change of condition is relatively insensitive to the ambient conditions, which might otherwise cause the indicated change in the period of time to deviate substantially from the actual amount of time that has passed. For example, phase change indicators which rely on the evaporation of a chemical component in the indicator to the atmosphere may be particularly sensitive to ambient temperature because of the effect this has on the rate of evaporation.

The transdermal patch timer may be a single-use device. The transdermal patch timer may not be not integrally formed with the transdermal patch.

The transdermal patch timer may comprise a dye reservoir. The transdermal patch timer may comprise a porous membrane. The porous membrane may be adjacent to the dye reservoir. The porous membrane may be configured to allow dye to flow through the porous membrane at a predetermined rate.

The transdermal patch timer may comprise an activation member. The activation member configured to initiate measurement of time by the timer in response to application of a transdermal patch to a patient, which may be achieved manually in response to user input. The initiation of the measurement may be irrevocable or irreversible.

The activation member may be configured to initiate measurement by enabling fluid flow from the dye reservoir to the porous membrane. The activation member may comprise a barrier situated between the dye reservoir and the indicator. The barrier may be situated between the dye reservoir and the porous membrane. The barrier may be a meltable barrier. The barrier may be configured to melted by body heat. The barrier may be an adhesive or wax.

Alternatively, the activation member may be configured to initiate measurement by enabling fluid flow between the indicator and an atmosphere surrounding the transdermal patch timer.

According to a further aspect there may be provided a structure according to claim 10. The carrier layer may comprise registration features, or fiducial markers, for registering the plurality of transdermal patch timers with a corresponding plurality of transdermal patches.

According to a further aspect there is provided a transdermal patch comprising a transdermal patch timer described herein. The transdermal patch may comprise a cover layer. The transdermal patch may comprise a reservoir for active agent adjacent to the cover layer. The transdermal patch may comprise an adhesive for adhering the cover layer to a user's skin. The transdermal patch may comprise a liner layer configured to protect the adhesive layer when in storage.

The transdermal patch timer and liner layer may be arranged such that removal of the liner layer from the transdermal patch initiates the transdermal patch timer.

The transdermal patch may comprise an active agent in the reservoir. The active agent comprises fentanyl.

According to a further aspect there is provided a method of manufacturing a transdermal patch according to claim 15. The method may comprise applying a transdermal patch timer to a cover layer of a transdermal patch. The method may comprise aligning a first structure with a second structure. The first structure may comprise a carrier layer. The second structure may comprise a carrier layer. The first structure may comprise a plurality of transdermal patches. The second structure may comprise a plurality of the transdermal patch timers. The method may comprise applying each of the transdermal patch timers with a corresponding transdermal patch.

According to a further aspect, not belonging to the invention, there is provided a method of using a transdermal patch. The method may comprise adhering the transdermal patch to the user. The method may comprise activating a transdermal patch timer on the transdermal patch. The method may comprise removing the transdermal patch from the skin of a patient in response to observing that the transdermal patch timer shows that a predetermined period of time has elapsed.

Any reference herein to a patient may equally refer to another type of user, or vice versa. In general terms a patient is a wearer of the transdermal patch.

### Brief Description of Figures

Embodiments of the present disclosure will now be described by way of example and with reference to the accompanying drawings in which:
Figure 1 illustrates a transdermal patch timer;
Figures 2a, 2b and 2c illustrate various views of a transdermal patch comprising the transdermal patch timer of Figure 1;
Figure 3 illustrates a flow diagram for a method of manufacturing a transdermal patch;
Figure 5 illustrates another transdermal patch timer;
Figure 6 illustrates a transdermal patch comprising the transdermal patch timer of Figure 5;
Figure 7 illustrates a further transdermal patch timer; and
Figure 8 illustrates a transdermal patch comprising the transdermal patch timer of Figure 7.

### Description of Examples

The present invention relates to a transdermal patch timer for applying to a transdermal patch. The provision of a time indicator on the transdermal patch may provide a clear and unambiguous means for a patient or carer to ensure that the patient's transdermal patch is used or replaced in accordance with a prescription. Such an indicator may be easier for the patient or career to comprehend than, for example, a written instruction on the patch indicating a time or date that the patch was applied or should be changed. In this way, the incidents of underdose or overdose using the transdermal patch may be eliminated or reduced.

A further contribution according to some aspects relates to the provision of the transdermal patch timer as a separate means to the delivery of active agent using the transdermal patch. That is, the progression of the time indicator may be independent from the progression of delivery of the active agent to the patient. In this way, technical difficulties, and associated regulatory difficulties, with the potential for interaction between the active agent and the indicator may be avoided. In addition, by the provision of the independence of the transdermal patch timer from the transdermal patch active agent delivery system, the process of manufacturing a transdermal patch comprising a transdermal patch timer may be simplified or otherwise improved.

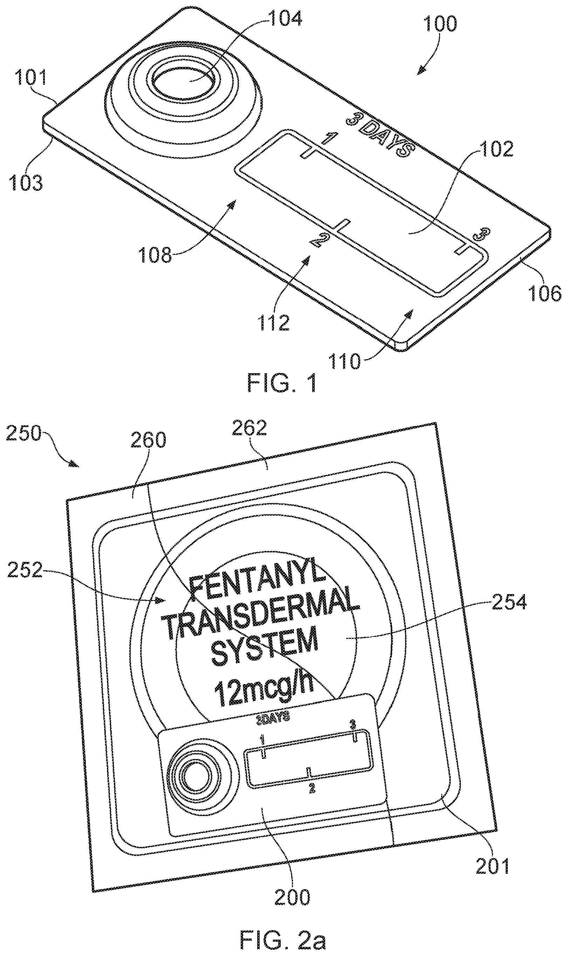

Figure 1 illustrates a transdermal patch timer 100. The unit 100 comprises an indicator 102 and an activation member 104, each provided on a planar layer 106.

In this example, the indicator 102 is in the form of a display. The display provides a visual indication of a period of time that has elapsed. The visual indication is provided by a change in appearance of the display as a function of time. In particular, in this example, the display provides a progress bar. The extent of a bar on the progress bar indicates a period of time that has elapsed. The activation member 104 controls the initiation of the time.

In this example, the activation member 104 is provided by a button. The button controls whether a dye can pass from a dye reservoir (not shown) situated under the button to reach the display. For example, patient actuation of the activation member 104 may cause the rupture of a membrane between the dye reservoir and the display. The display may be provided by a porous material that is configured to allow dye to flow through the material. The rate of passage of the dye from a first end 108 of the display, proximal to the dye reservoir, towards a second end 110 of the display, distal from the dye reservoir, as a function of time, is predetermined. In this way, markers 112 may be provided along the indicator 102 between the first end 108 and the second end 110 to indicate the passage of time since the initiation of the timer using the activation member 104. It will be appreciated that, in this example, the transdermal patch timer is a single-use device because the indicator cannot be reset.

The indicator 102 and the activation member 104 are provided on a first surface 101 of the transdermal patch timer 100. The transdermal patch timer 100 further comprises a second surface 103, which opposes the first surface 101. An adhesive (not shown) may be provided on the second surface 103 in order to adhere the transdermal patch timer 100 to the cover of a transdermal patch. The adhesive is an example of an attachment mechanism that is configured to couple the transdermal patch timer to the transdermal patch.

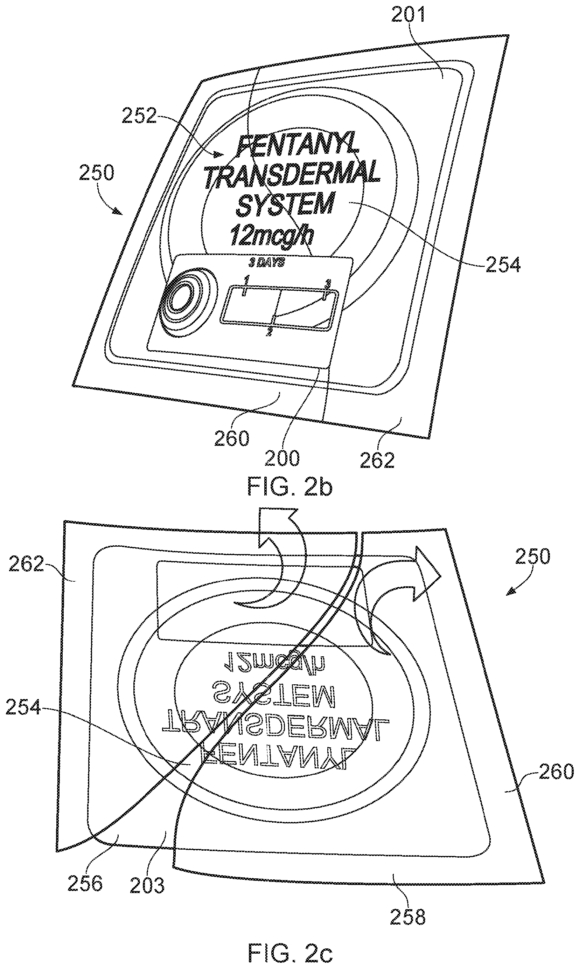

Figures 2a, 2b and 2c illustrate various views of a transdermal patch 250 comprising a transdermal patch timer 200.

Figures 2a and 2b both illustrate isometric perspective views of an external surface (when in use) 201 of the transdermal patch 250. The transdermal patch timer 200, which is similar to that described previously with reference to Figure 1, is provided on the first surface 201 of the transdermal patch 250. The first surface 201 provides an outer surface of the transdermal patch 250 when in use. An indication 252 of the active agent (no shown) within the transdermal patch 250 is also provided on the first surface 201 of the transdermal patch 250 in this example.

The transdermal patch 250 comprises a four-layer structure including, in order;
- the transdermal patch timer 200.
- the cover layer with the first surface 201 (shown in figures 2a and 2b) and an opposing second surface 203 (shown in figure 2c).
- an application layer, provided on the second surface 203 of the cover layer, comprising adhesive 256 surrounding an active agent reservoir 254.
- a liner layer 258.

Such a structure provides that the indicator of the transdermal patch timer 200 is isolated from the active agent reservoir 254 of the transdermal patch 250. The indicator is configured to operate independently from the delivery of active agent to the patient in the active agent reservoir 254. Such an arrangement may overcome potential difficulties cause by interaction between the active agent and the indicator means, which in this example is provided by a chemical that changes condition over time.

The cover layer may be provided by an EVA polymer layer.

An active agent, such as fentanyl, may be provided in the active agent reservoir. The adhesive may be provided by a conventional adhesive used in transdermal patches.

The liner layer 258 may be provided by a siliconized PET layer. The liner layer 258 comprises a first liner portion 260 and a second liner portion 262. The liner layer 258 is configured to protect the adhesive layer and retain the active agent within the reservoir 254 when the transdermal patch 250 is in storage. The adhesive layer 256 and the reservoir 254 may be exposed by pulling away the first and second portions 260, 262 of the liner layer 258 when the transdermal patch 250 is to be applied to a patient. The provision of the separate first liner portion 260 and second liner portion 262 may assist in removal of the liner layer 258 in some examples.

It will be appreciated that other transdermal patch structures may be provided in an alternative to the example described with reference to Figures 2a to 2c.

The transdermal patch may be manufactured.

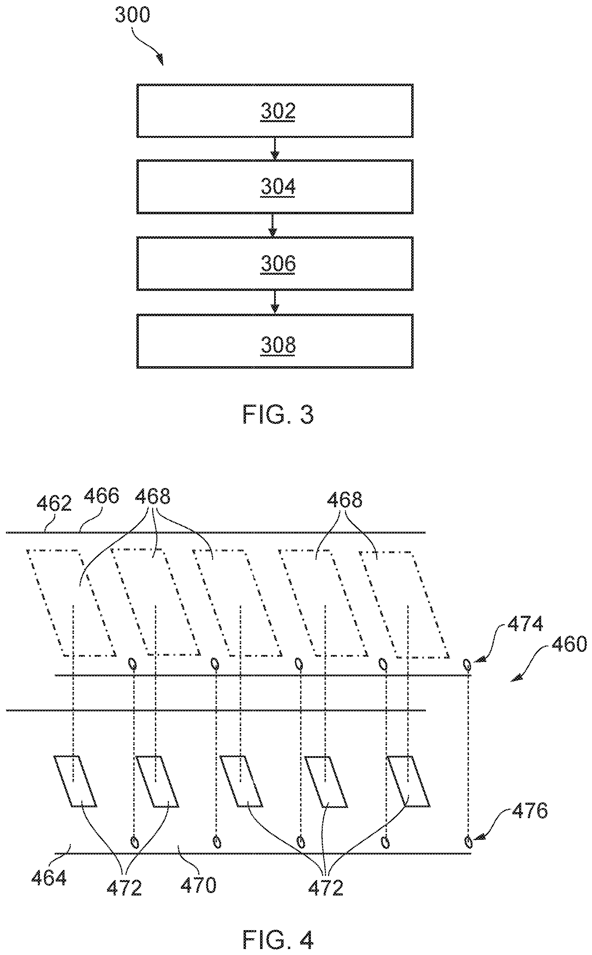

Figure 3 illustrates a flow diagram for a method of using a transdermal patch such as that described previously with reference to Figures 2a to 2c.

The method 300 comprises:
- exposing 302, on a second surface of the transdermal patch, active agent for delivery to the patient transdermally and exposing adhesive for adhering the transdermal patch to the patient's skin;
- adhering 304 the transdermal patch to the patient's skin to initiate delivery of the active agent;
- activating 306 a transdermal patch timer on the transdermal patch;
- removing 308 the transdermal patch from the skin of a patient in response to a patient observing that the transdermal patch timer shows that a predetermined period of time has elapsed.

The transdermal patch timer may be activated manually by a patient or carer when adhering 304 the transdermal patch to the patient's skin. In this way, the initiation of the transdermal patch timer is synchronized with the initiation of delivery of the active agent. In some examples, the act of exposing the active agent or adhesive, for example by the removal of a liner, may cause initiation of the timer. For example, in relation to Figures 1 and 2a to 2c, the liner may be connected to the membrane that separates the dye from the display so that removal of the liner causes at least a partial rupture of the membrane and therefore initiates the timer.

It will be appreciated from the foregoing description of Figures 1, 2a, 2b and 2c that, in some examples, the transdermal patch may not be integrally formed with the transdermal patch timer. Figure 4 illustrates an intermediate structure 460 that may be provided during the manufacture of a transdermal patch comprising a transdermal patch timer. Figure 4 shows a first structure 462 and a second structure 464.

The first structure 462 comprises a first carrier layer 466 and a plurality of transdermal patches 468. The plurality of transdermal patches 468 may be provided on the first structure 462 such that respective second surfaces of the transdermal patches 468, as described previously with reference to Figures 2a-2c, may be removably attached to the carrier layer 466. Each transdermal patch 468 may comprise a cover layer, an application layer and a liner layer as described previously with reference to Figures 2a-2c. The liner layer may be formed from, or connected to, the first carrier layer 462. A first surface of the cover layer of the plurality of transdermal patches 468 faces the second structure 464.

The second structure comprises a second carrier layer 470 and a plurality of transdermal patch timers 472. The plurality of transdermal patch timers 472 may be provided on the second structure 464 such that a first surface of the transdermal patch timers 472, as described previously with reference to Figure 1, may be removably attached to the carrier layer 474. This may be achieved by a temporary adhesive, for example. The second surfaces of the plurality of transdermal patch timers 472, which provide an attachment means for coupling the transdermal patch timers 472 to the plurality of transdermal patches 464, face the first structure 462.

During a manufacture process, the first structure 462 may be aligned with the second structure 464 such that each transdermal patch timer 472 corresponds to, and is aligned with, a respective transdermal patch 468. A first set of registration features 474 may be provided on the first structure 462 for registration with a corresponding second set of registration features 476 on the second structure 464. The registration features may be physical registration features, such as notches for holes in the respective first and second structures 462, 464. Alternatively, the registration features 474, 476 may be provided by fiducial markers. In this example, the first and second sets of registration features 474, 476 are each provided along respective edges of the first and second structures 462, 464.

During the process of manufacture, the first structure 462 may be brought into contact with the second structure 464 such that the transdermal patch timers 472 are engaged with the corresponding transdermal patches 468. The structures may be brought together at a single point which is moved along the respective carriers 466, 470 so that the corresponding transdermal patch timers 472 and transdermal patches 468 are brought together in turn. Alternatively, an area of the first structure 462 may be brought into contact with a corresponding area of the second structure 464 such that a plurality of the transdermal patch timers 472 are engaged with a corresponding plurality of the transdermal patches 468 simultaneously.

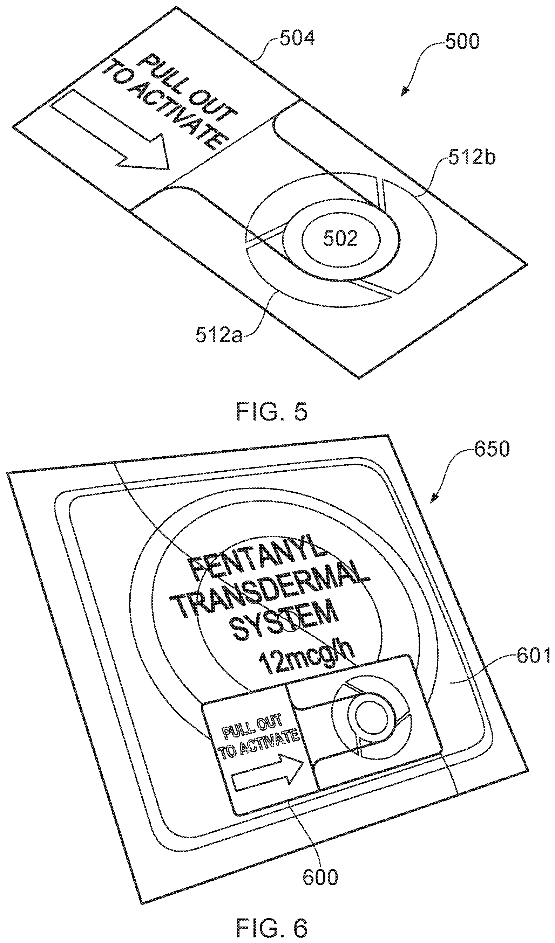

Figure 5 illustrates another transdermal patch timer 500. The transdermal patch timer 500 comprises an indicator 502 which changes colour in response to exposure to the atmosphere. The indicator 502 may be a carbonate indicator or another indicator that changes colour with CO₂ exposure over time. The indicator 502 is provided as a central dot that is adjacent to a first region 512a and a second region 512b. The first region 512a has a colour that matches a colour which the indicator 502 is calibrated to adopt after exposure to the atmosphere for a first period of time (for example, 3 days). The second region 512b has a colour that matches a colour which the indicator 502 is calibrated to adopt after exposure to the atmosphere for a second period of time (for example, 20 days).

The transdermal patch timer 500 further comprises an activation member 504. The activation member 504 is configured to initiate the timer 500 by enabling fluid flow between the indicator 502 and the atmosphere surrounding the transdermal patch timer 500. The indicator 502 may be activated by a user peeling-off a protective film covering the central dot. Over time, the central dot will then change colour to match the different time periods shown in the outer ring by the first and second regions 512a, 512b.

Figure 6 illustrates another transdermal patch 650 comprising a transdermal patch timer 600. The transdermal patch 650 is similar to that described previously with reference to Figures 2a to 2c except that the transdermal patch timer provided on a first surface 601 of the transdermal patch 600 is similar to that described previously with reference to Figure 5.

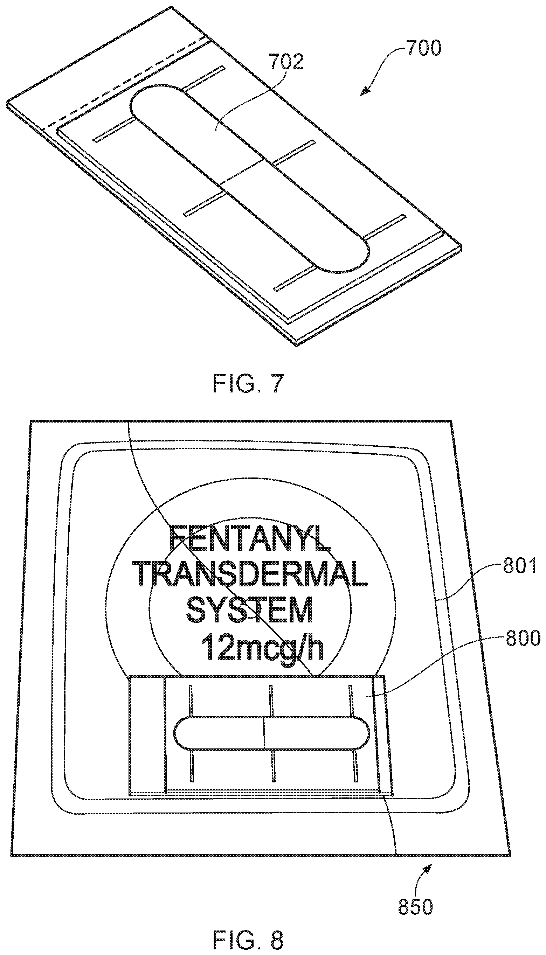

Figure 7 illustrates a further transdermal patch timer 700. As in the example described previously with reference to Figures 2a, 2b and 2c, the transdermal patch timer 700 comprises an indicator 702 provided by a display in which dye travels through a porous membrane at a known rate.

The transdermal patch timer 700 further comprises an activation member (not shown). The activation member is configured to initiate the timer 500 by enabling dye to flow from a dye reservoir (not shown) to the display. The activation member is provided by a barrier, which may be an adhesive or wax barrier, which is configured to melt when the transdermal patch timer 700 is heated. In this way, the dye is released from the dye reservoir to the display by the adhesive barrier melting due to proximity to body heat when a transdermal patch comprising the transdermal patch timer 700 is applied to a patient's skin. The melting temperature may be selected to be substantially above room temperature but below typical skin temperature. The melting temperature of the barrier may be between 25 °C and 34 °C, and more preferably between 28 °C and 30 °C.

Figure 8 illustrates another transdermal patch 850 comprising a transdermal patch timer 800. The transdermal patch 850 is similar to that described previously with reference to Figures 2a to 2c except that the transdermal patch timer provided on a first surface 801 of the transdermal patch 800 is similar to that described previously with reference to Figure 7.

Although each of the embodiments described above relates to a transdermal patch timer comprising a visual display in which the passage of time is indicated as a result of the change in condition of a chemical, it will be appreciated that another indicator means may be provided. The change of condition may be achieved via a chemical reaction or phase change, for example. In general terms, an indication that a predetermined period of time has elapsed may be provided visually, audibly, by smell, by tactile feedback or by other electronic means. Similarly, although a visual output is provided by a chemical indicator, as an alternative, the visual output may be provided by an electronic timing circuit. The visual output may be an electronic display (such as a light emitting diode (LED) or liquid crystal display (LCD)) or by an indicator light, for example. In this way, a timing circuit may indicate that a predetermined period of time has elapsed following activation of the timing circuit. As a further alternative, the output of the timing circuit may be provided audibly, for example by a buzzer or speaker. As a further alternative, the output may be provided by haptic feedback to alert the patient that the period of time has elapsed. Other electronic means for providing an output of a timing device may include transmitting a signal from the transdermal patch timer in response to the predetermined period of time having elapsed. The transmitted signal may be provided to a separate device, such as a personal electronic device (for example, a patient's mobile telephone or computer). In this way, the separate device may provide the indication that a pre-determined period of time has elapsed. In such examples, a transmitter of the transdermal patch is configured to, in effect, indicate whether a pre-determined period of time has elapsed.

Examples in which a period of time is determined to have elapsed using a smell indication may involve releasing a chemical or enabling a chemical reaction in response to the pre-determined period of time having elapsed.

As a further alternative, although the embodiments described previously with reference to figures 1, 2 and 5 to 8 each relate to examples in which the transdermal patch timer is configured to be applied to a transdermal patch using an adhesive layer, it will be appreciated that other engagement means may be provided. For example, the transdermal patch timer may be sewn on to the transdermal patch, using a thread for example. As a further alternative, the transdermal patch timer may be integrally formed with the transdermal patch. The scope of protection of the current invention is defined by the appended claims.

## Claims

1. A transdermal patch timer (100, 200, 500, 600) comprising:
an indicator (102, 502) configured to indicate whether a predetermined period of time has elapsed; and
an attachment mechanism (256) configured to couple the transdermal patch timer (100, 200, 500, 600) to a transdermal patch (250, 650), **characterised in that** the indicator (102, 502) is a carbon dioxide indicator, and comprises a chemical that changes condition, via a chemical reaction, over time or after the predetermined period of time has elapsed.

2. The transdermal patch timer (100, 200, 500, 600) of any preceding claim, in which the attachment mechanism (256) comprises an adhesive layer configured to adhere the transdermal patch timer (100, 200, 500, 600) to the transdermal patch (250, 650).

3. The transdermal patch timer (100, 200, 500, 600) of any preceding claim, in which the indicator (102, 502) is arranged on the transdermal patch timer (100, 200, 500, 600) to be isolated from an active agent reservoir (254) of the transdermal patch (250, 650).

4. The transdermal patch timer (100, 200, 500, 600) of any preceding claim, in which the indicator (102, 502) comprises a visual indicator configured to undergo a change in appearance over time or after the predetermined period of time has elapsed, optionally in which the visual indicator is configured to change colour over time or after the predetermined period of time has elapsed.

5. The transdermal patch timer (100, 200, 500, 600) of any preceding claim, comprising a dye reservoir and a porous membrane adjacent to the dye reservoir, wherein the porous membrane is configured to allow dye to flow through the porous membrane at a predetermined rate.

6. The transdermal patch timer (100, 200, 500, 600) of any preceding claim, comprising an activation member (104, 504) configured to initiate measurement of the time by the indicator (102, 502) in response to application of a transdermal patch (250, 650) to a patient.

7. The transdermal patch timer (100, 200, 500, 600) of claim 6, in which the activation member (104, 504) is configured to initiate measurement by enabling fluid flow from the dye reservoir to the indicator (102, 502), optionally in which the activation member (104, 504) comprises a meltable barrier situated between the dye reservoir to the indicator (102, 502).

8. The transdermal patch timer (100, 200, 500, 600) of claim 6, in which the activation member (104, 504) is configured to initiate measurement by enabling fluid flow between the indicator (102, 502) and an atmosphere surrounding the transdermal patch timer (100, 200, 500, 600).

9. The transdermal patch timer (100, 200, 500, 600) of any of claims 6 to 8, in which the initiation of the measurement is irreversible.

10. A structure **characterised by** comprising a carrier layer and a plurality of the transdermal patch timers (100, 200, 500, 600) of claim 1, optionally in which the carrier layer comprises registration features for registering the plurality of transdermal patch timers (100, 200, 500, 600) with a corresponding plurality of transdermal patches (250, 650).

11. A transdermal patch (250, 650) **characterised by** comprising the transdermal patch timer (100, 200, 500, 600) of any of claims 1 to 8.

12. The transdermal patch (250, 650) of claim 11, further comprising:
a cover layer;
a reservoir (254) for active agent adjacent to the cover layer;
an adhesive for adhering the cover layer to a user's skin; and
a liner layer (258) configured to protect the adhesive layer when in storage.

13. The transdermal patch (250, 650) of claim 12, in which the transdermal patch timer (100, 200, 500, 600) and liner layer (258) are arranged such that removal of the liner layer (258) from the transdermal patch (250, 650) initiates the transdermal patch timer (100, 200, 500, 600).

14. The transdermal patch (250, 650) of claim 12 or claim 13, comprising active agent in the reservoir (254), optionally wherein the active agent comprises fentanyl.

15. A method of manufacturing a transdermal patch (250, 650) **characterised by** comprising applying a transdermal patch timer (100, 200, 500, 600) of claim 1 to a transdermal patch (250, 650), optionally further comprising:
aligning (i) a first structure comprising a carrier layer (466) and a plurality of the transdermal patch timers (100, 200, 500, 600) with (ii) a second structure comprising a carrier layer (470) and a plurality of transdermal patches (250, 650), and
applying each of the transdermal patch timers (100, 200, 500, 600) with a corresponding transdermal patch (250, 650).

## Patentansprüche

1. Zeitgeber (100, 200, 500, 600) für ein transdermales Pflaster, umfassend:
eine Anzeigeeinrichtung (102, 502), die dazu konfiguriert ist, anzugeben, ob eine vorbestimmte Zeitspanne verstrichen ist; und
einen Befestigungsmechanismus (256), der dazu konfiguriert ist, den Zeitgeber (100, 200, 500, 600) für ein transdermales Pflaster an ein transdermales Pflaster (250, 650) zu koppeln, **dadurch gekennzeichnet, dass** die Anzeigeeinrichtung (102, 502) eine Kohlendioxid-Anzeigeeinrichtung ist und eine Chemikalie umfasst, die den Zustand mittels einer chemischen Reaktion im Laufe der Zeit oder nach Verstreichen der vorbestimmten Zeitspanne ändert.

2. Zeitgeber (100, 200, 500, 600) für ein transdermales Pflaster nach einem vorhergehenden Anspruch, wobei der Befestigungsmechanismus (256) eine Klebeschicht umfasst, die dazu konfiguriert ist, den Zeitgeber (100, 200, 500, 600) für ein transdermales Pflaster auf das transdermale Pflaster (250, 650) zu kleben.

3. Zeitgeber (100, 200, 500, 600) für ein transdermales Pflaster nach einem vorhergehenden Anspruch, wobei die Anzeigeeinrichtung (102, 502) auf dem Zeitgeber (100, 200, 500, 600) für ein transdermales Pflaster so angeordnet ist, dass sie von einem Wirkstoffreservoir (254) des transdermalen Pflasters (250, 650) getrennt ist.

4. Zeitgeber (100, 200, 500, 600) für ein transdermales Pflaster nach einem vorhergehenden Anspruch, wobei die Anzeigeeinrichtung (102, 502) eine visuelle Anzeigeeinrichtung umfasst, die dazu konfiguriert ist, eine Änderung des Erscheinungsbilds im Laufe der Zeit oder nach Verstreichen der vorbestimmten Zeitspanne zu erfahren, wobei die visuelle Anzeigeeinrichtung optional dazu konfiguriert ist, die Farbe im Laufe der Zeit oder nach Verstreichen der vorbestimmten Zeitspanne zu ändern.

5. Zeitgeber (100, 200, 500, 600) für ein transdermales Pflaster nach einem vorhergehenden Anspruch, umfassend ein Farbstoffreservoir und eine poröse Membran benachbart zu dem Farbstoffreservoir, wobei die poröse Membran dazu konfiguriert ist, es dem Farbstoff zu erlauben, mit einem vorbestimmten Durchsatz durch die poröse Membran zu strömen.

6. Zeitgeber (100, 200, 500, 600) für ein transdermales Pflaster nach einem vorhergehenden Anspruch, umfassend ein Aktivierungselement (104, 504), das dazu konfiguriert ist, eine Messung der Zeit durch die Anzeigeeinrichtung (102, 502) als Reaktion auf das Anbringen eines transdermalen Pflasters (250, 650) an einem Patienten zu initiieren.

7. Zeitgeber (100, 200, 500, 600) für ein transdermales Pflaster nach Anspruch 6, wobei das Aktivierungselement (104, 504) dazu konfiguriert ist, eine Messung zu initiieren, indem ein Fluidstrom von dem Farbstoffreservoir zu der Anzeigeeinrichtung (102, 502) ermöglicht wird, wobei das Aktivierungselement (104, 504) optional eine schmelzbare Barriere umfasst, die sich zwischen dem Farbstoffreservoir zu der Anzeigeeinrichtung (102, 502) befindet.

8. Zeitgeber (100, 200, 500, 600) für ein transdermales Pflaster nach Anspruch 6, wobei das Aktivierungselement (104, 504) dazu konfiguriert ist, eine Messung zu initiieren, indem ein Fluidstrom zwischen der Anzeigeeinrichtung (102, 502) und einer Atmosphäre, die den Zeitgeber (100, 200, 500, 600) für ein transdermales Pflaster umgibt, ermöglicht wird.

9. Zeitgeber (100, 200, 500, 600) für ein transdermales Pflaster nach einem der Ansprüche 6 bis 8, wobei die Initiierung der Messung irreversibel ist.

10. Struktur, **dadurch gekennzeichnet, dass** sie eine Trägerschicht und eine Vielzahl der Zeitgeber (100, 200, 500, 600) für ein transdermales Pflaster nach Anspruch 1 umfasst, wobei die Trägerschicht optional Registrierungsmerkmale zum Registrieren der Vielzahl von Zeitgebern (100, 200, 500, 600) für ein transdermales Pflaster mit einer entsprechenden Vielzahl von transdermalen Pflastern (250, 650) umfasst.

11. Transdermales Pflaster (250, 650), **dadurch gekennzeichnet, dass** es den Zeitgeber (100, 200, 500, 600) für ein transdermales Pflaster nach einem der Ansprüche 1 bis 8 umfasst.

12. Transdermales Pflaster (250, 650) nach Anspruch 11, ferner umfassend:
eine Deckschicht;
ein Reservoir (254) für Wirkstoff benachbart zu der Deckschicht;
einen Klebstoff zum Kleben der Deckschicht an die Haut eines Benutzers; und
eine Schutzschicht (258), die dazu konfiguriert ist, die Klebstoffschicht während der Lagerung zu schützen.

13. Transdermales Pflaster (250, 650) nach Anspruch 12, wobei der Zeitgeber (100, 200, 500, 600) für ein transdermales Pflaster und die Schutzschicht (258) so angeordnet sind, dass ein Entfernen der Schutzschicht (258) von dem transdermalen Pflaster (250, 650) den Zeitgeber (100, 200, 500, 600) für ein transdermales Pflaster initiiert.

14. Transdermales Pflaster (250, 650) nach Anspruch 12 oder Anspruch 13, umfassend einen Wirkstoff in dem Reservoir (254), optional wobei der Wirkstoff Fentanyl umfasst.

15. Verfahren zum Herstellen eines transdermalen Pflasters (250, 650), **dadurch gekennzeichnet, dass** es Anbringen eines Zeitgebers (100, 200, 500, 600) für ein transdermales Pflaster nach Anspruch 1 an einem transdermalem Pflaster (250, 650) umfasst, optional ferner umfassend:
Ausrichten (i) einer ersten Struktur, die eine Trägerschicht (466) und eine Vielzahl der Zeitgeber (100, 200, 500, 600) für ein transdermales Pflaster umfasst, auf (ii) eine zweite Struktur, die eine Trägerschicht (470) und eine Vielzahl von transdermalen Pflastern (250, 650) umfasst, und
Anbringen jedes der Zeitgeber (100, 200, 500, 600) für ein transdermales Pflaster mit einem entsprechenden transdermalen Pflaster (250, 650).

## Revendications

1. Temporisateur de timbre transdermique (100, 200, 500, 600) comprenant :
un indicateur (102, 502) conçu pour indiquer si une période de temps prédéterminée s'est écoulée ; et
un mécanisme de fixation (256) conçu pour coupler le temporisateur de timbre transdermique (100, 200, 500, 600) à un timbre transdermique (250, 650), **caractérisé en ce que** l'indicateur (102, 502) est un indicateur de dioxyde de carbone, et comprend un produit chimique qui change d'état, par une réaction chimique, au fil du temps ou après écoulement de la période de temps prédéterminée.

2. Temporisateur de timbre transdermique (100, 200, 500, 600) d'une quelconque revendication précédente, dans lequel le mécanisme de fixation (256) comprend une couche adhésive conçue pour coller le temporisateur de timbre transdermique (100, 200, 500, 600) au timbre transdermique (250, 650).

3. Temporisateur de timbre transdermique (100, 200, 500, 600) d'une quelconque revendication précédente, dans lequel l'indicateur (102, 502) est agencé sur le temporisateur de timbre transdermique (100, 200, 500, 600) pour être isolé d'un réservoir d'agent actif (254) du timbre transdermique (250, 650).

4. Temporisateur de timbre transdermique (100, 200, 500, 600) d'une quelconque revendication précédente, dans lequel l'indicateur (102, 502) comprend un indicateur visuel conçu pour subir un changement d'apparence au fil du temps ou après écoulement de la période de temps prédéterminée, éventuellement dans lequel l'indicateur visuel est conçu pour changer de couleur au fil du temps ou après écoulement de la période de temps prédéterminée.

5. Temporisateur de timbre transdermique (100, 200, 500, 600) d'une quelconque revendication précédente, comprenant un réservoir de colorant et une membrane poreuse adjacente au réservoir de colorant, dans lequel la membrane poreuse est conçue pour permettre à un colorant de s'écouler à travers la membrane poreuse à une vitesse prédéterminée.

6. Temporisateur de timbre transdermique (100, 200, 500, 600) d'une quelconque revendication précédente, comprenant un élément d'activation (104, 504) conçu pour initier la mesure du temps par l'indicateur (102, 502) en réponse à l'application d'un timbre transdermique (250, 650) à un patient.

7. Temporisateur de timbre transdermique (100, 200, 500, 600) de la revendication 6, dans lequel l'élément d'activation (104, 504) est conçu pour initier la mesure en permettant l'écoulement de fluide du réservoir de colorant vers l'indicateur (102, 502), éventuellement dans lequel l'élément d'activation (104, 504) comprend une barrière fusible située entre le réservoir de colorant et l'indicateur (102, 502).

8. Temporisateur de timbre transdermique (100, 200, 500, 600) de la revendication 6, dans lequel l'élément d'activation (104, 504) est conçu pour initier la mesure en permettant l'écoulement de fluide entre l'indicateur (102, 502) et une atmosphère entourant le temporisateur de timbre transdermique (100, 200, 500, 600).

9. Temporisateur de timbre transdermique (100, 200, 500, 600) de l'une quelconque des revendications 6 à 8, dans lequel l'initiation de la mesure est irréversible.

10. Structure **caractérisée en ce qu'**elle comprend une couche de support et une pluralité de temporisateurs de timbre transdermique (100, 200, 500, 600) de la revendication 1, éventuellement dans laquelle la couche de support comprend des éléments d'enregistrement pour enregistrer la pluralité de temporisateurs de timbre transdermique (100, 200, 500, 600) avec une pluralité correspondante de timbres transdermiques (250, 650).

11. Timbre transdermique (250, 650) **caractérisé en ce qu'**il comprend le temporisateur de timbre transdermique (100, 200, 500, 600) de l'une quelconque des revendications 1 à 8.

12. Timbre transdermique (250, 650) de la revendication 11, comprenant en outre :
une couche de couverture ;
un réservoir (254) pour un agent actif adjacent à la couche de couverture ;
un adhésif pour coller la couche de couverture à la peau d'un utilisateur ; et
une couche de revêtement (258) conçue pour protéger la couche adhésive lors du stockage.

13. Timbre transdermique (250, 650) de la revendication 12, dans lequel le temporisateur de timbre transdermique (100, 200, 500, 600) et la couche de revêtement (258) sont agencés de sorte que le retrait de la couche de revêtement (258) du timbre transdermique (250, 650) initie le temporisateur de timbre transdermique (100, 200, 500, 600).

14. Timbre transdermique (250, 650) de la revendication 12 ou la revendication 13, comprenant un agent actif dans le réservoir (254), dans lequel éventuellement l'agent actif comprend du fentanyl.

15. Procédé de fabrication d'un timbre transdermique (250, 650), **caractérisé en ce qu'**il comprend l'application d'un temporisateur de timbre transdermique (100, 200, 500, 600) de la revendication 1 à un timbre transdermique (250, 650), comprenant en outre éventuellement :
l'alignement (i) d'une première structure comprenant une couche de support (466) et une pluralité de temporisateurs de timbre transdermique (100, 200, 500, 600) avec (ii) une seconde structure comprenant une couche de support (470) et une pluralité de timbres transdermiques (250, 650), et
l'application de chacun des temporisateurs de timbre transdermique (100, 200, 500, 600) avec un timbre transdermique correspondant (250, 650).
